# EUROPEAN PATENT SPECIFICATION

(11) **EP 3 477 589 B1**
(45) Date of publication and mention of the grant of the patent: **19.08.2020**
(21) Application number: 18202742.5
(22) Date of filing: 26.10.2018
(51) Int. Cl.: G06T 11/00, G06T 7/00, G16H 30/40, G16H 50/20

(54) **METHOD OF PROCESSING MEDICAL IMAGE, AND MEDICAL IMAGE PROCESSING APPARATUS PERFORMING THE METHOD**
VERFAHREN ZUR VERARBEITUNG MEDIZINISCHER BILDER UND VORRICHTUNG ZUR VERARBEITUNG MEDIZINISCHER BILDER ZUR DURCHFÜHRUNG DES VERFAHRENS
PROCÉDÉ DE TRAITEMENT D'IMAGE MÉDICALE, ET APPAREIL DE TRAITEMENT D'IMAGE MÉDICALE METTANT EN UVRE CE PROCÉDÉ

(30) Priority: 26.10.2017 KR 20170140317
(43) Date of publication of application: 01.05.2019
(73) Proprietor: Samsung Electronics Co., Ltd., Gyeonggi-do 16677 (KR)
(72) Inventor: Lee, Dong-jae, Seoul (KR); Oh, Hyun-hwa, Gyeonggi-do (KR); Kim, Se-min, Gyeonggi-do (KR); Song, Jeong-yong, Gyeonggi-do (KR); Lee, Hyun-jung, Gyeonggi-do (KR)
(74) Representative: Jacobs, Bart

(56) References cited:
- JP-A- 2011 182 960
- US-A1- 2017 357 844
- US-B1- 8 879 813
- ANDRE ESTEVA ET AL: "Dermatologist-level classification of skin cancer with deep neural networks", NATURE, vol. 542, no. 7639, 25 January 2017 (2017-01-25), pages 115-118, XP055536881, London ISSN: 0028-0836, DOI: 10.1038/nature21056
- CHRISTOPHER BOWLES ET AL: "Modelling the progression of Alzheimer's disease in MRI using generative adversarial networks", MEDICAL IMAGING 2018: IMAGE PROCESSING, 2 March 2018 (2018-03-02), page 55, XP055536880, DOI: 10.1117/12.2293256 ISBN: 978-1-5106-1638-7
- Xin Yi ET AL: "Generative Adversarial Network in Medical Imaging: A Review", arXiv, 19 September 2018 (2018-09-19), XP055536878, Retrieved from the Internet: URL:https://arxiv.org/pdf/1809.07294.pdf [retrieved on 2018-12-19]
- SHIN HOO-CHANG ET AL: "Medical Image Synthesis for Data Augmentation and Anonymization Using Generative Adversarial Networks", 12 September 2018 (2018-09-12), INTERNATIONAL CONFERENCE ON COMPUTER ANALYSIS OF IMAGES AND PATTERNS. CAIP 2017: COMPUTER ANALYSIS OF IMAGES AND PATTERNS; [LECTURE NOTES IN COMPUTER SCIENCE; LECT.NOTES COMPUTER], SPRINGER, BERLIN, HEIDELBERG, PAGE(S) 1 - 11, XP047485123, ISBN: 978-3-642-17318-9 [retrieved on 2018-09-12]
- GRIGORY ANTIPOV ET AL: "Face Aging With Conditional Generative Adversarial Networks", ARXIV.ORG, CORNELL UNIVERSITY LIBRARY, 201 OLIN LIBRARY CORNELL UNIVERSITY ITHACA, NY 14853, 7 February 2017 (2017-02-07), XP080746931, DOI: 10.1109/ICIP.2017.8296650

## Description

### BACKGROUND

### 1. Field

The disclosure relates to a medical image processing method for obtaining an additional image or additional information by analyzing a medical image obtained by a medical imaging apparatus, and a medical image processing apparatus performing the medical image processing method.

### 2. Description of the Related Art

Medical imaging apparatuses are equipment for capturing an image of an internal structure of an object such as a human body. Medical imaging apparatuses are noninvasive examination apparatuses that capture and process images of the structural details of a human body, internal tissues thereof, and fluid flow within the human body, and provide the processed images to a user. A user, such as a doctor or other medical professional, may diagnose a health status or a disease of a patient by using a medical image output from a medical imaging apparatus.

Examples of medical imaging apparatuses include an X-ray apparatus for obtaining an image by radiating an X-ray to an object and sensing an X-ray transmitted through the object, a magnetic resonance imaging (MRI) apparatus for providing a magnetic resonance (MR) image, a computed tomography (CT) apparatus, and an ultrasound diagnostic apparatus.

With recent developments in image processing technology, such as a computer aided detection (CAD) system and machine learning, medical imaging apparatuses may analyze an obtained medical image by using a computer, and thus detect an abnormal region, which is an abnormal part of an object, or generate a result of the analysis. The result of the analysis may assist with a doctor's diagnosis of a disease via the medical image and patient diagnosis.

In detail, to process this medical image, a CAD system that performs information processing based on artificial intelligence (AI) has been developed. The CAD system that performs information processing based on AI may be referred to as an AI system.

The AI system is a computer system configured to mimic or approximate human-level intelligence, and train itself to make determinations spontaneously to become smarter, in contrast to existing rule-based smart systems. Since a successful recognition rate of an AI system improves and the AI system more accurately understands a user's preferences the more it is used, existing rule-based smart systems are being gradually replaced by deep-learning AI systems.

AI technology includes machine learning (e.g., deep learning) and element technologies employing the machine learning.

The machine learning is an algorithm technology that self-classifies/learns the characteristics of input data, and each of the element technologies is a technology using a machine learning algorithm, such as deep learning, and includes technical fields such as linguistic understanding, visual understanding, deduction/prediction, knowledge representation, and operation control.

Various fields to which AI technology is applied are as follows. The linguistic understanding is a technique of recognizing a language/character of a human and applying/processing the language/character of a human, and includes natural language processing, machine translation, a conversation system, questions and answers, voice recognition/synthesis, and the like. The visual understanding is a technique of recognizing and processing an object like in human vision, and includes object recognition, object tracking, image search, human recognition, scene understanding, space understanding, image improvement, and the like. The deduction/prediction is a technology of logically performing deduction and prediction by determining information, and includes knowledge/probability-based deduction, optimization prediction, a preference-based plan, recommendation, and the like. The knowledge representation is a technique of automatically processing human experience information as knowledge data, and includes knowledge establishment (data generation/classification), knowledge management (data utilization), and the like. The operation control is a technique of controlling autonomous driving of a vehicle and motions of a robot, and includes motion control (navigation, collision avoidance, and driving), manipulation control (behavior control), and the like.

In AI systems, when the above-described visual understanding and deduction/prediction fields are applied to processing of a medical image, the medical image is more quickly and more accurately analyzed, thus helping a user, such as a doctor, to diagnose a patient via the medical image.

One such system is for example known from JP 2011-182960, which discloses the generation of a synthetic image depicting the predicted state of a photographed region of a patient after a certain time interval from the deformation fields obtained for the evolution of similar lesions and time intervals in previous patients. Another existing system is for example "Dermatologist-level classification of skin cancer with deep neural networks." Esteva, Andre, et al. Nature 542.7639 (2017): 115. Herein, it is disclosed that automated classification of skin lesions using images is a challenging task owing to the fine-grained variability in the appearance of skin lesions. Deep convolutional neural networks (CNNs) show potential for general and highly variable tasks across many fine-grained object categories. In this document, the authors demonstrate classification of skin lesions using a single CNN, trained end-to-end from images directly, using only pixels and disease labels as inputs. The authors train a CNN using a dataset of 129,450 clinical images-two orders of magnitude larger than previous datasets-consisting of 2,032 different diseases. The authors test its performance against 21 board-certified dermatologists on biopsy-proven clinical images with two critical binary classification use cases: keratinocyte carcinomas versus benign seborrheic keratoses; and malignant melanomas versus benign nevi. The first case represents the identification of the most common cancers; the second represents the identification of the deadliest skin cancer. The CNN achieves performance on par with all tested experts across both tasks, demonstrating an artificial intelligence capable of classifying skin cancer with a level of competence comparable to dermatologists. Outfitted with deep neural networks, mobile devices can potentially extend the reach of dermatologists outside of the clinic, it is projected that 6.3 billion smartphone subscriptions will exist by the year 2021 and can therefore potentially provide low-cost universal access to vital diagnostic care. Additionally, US 8,879,813 discloses the use of neural networks for comparing lesion images captured at different points in time.

### SUMMARY

Provided are a medical image processing method for obtaining at least one medical image from which a variation in a detected lesion over time may be predicted, when a lesion is included in or detected from an actual medical image obtained via imaging at a certain time point, and a medical image processing apparatus performing the medical image processing method.

Provided are a medical image processing method for obtaining information for use in accurately diagnosing a disease of a patient, via a deep neural network (DNN) that has trained a plurality of actual medical images including lesions, and a medical image processing apparatus performing the medical image processing method.

Additional aspects will be set forth in part in the description which follows and, in part, will be apparent from the description, or may be learned by practice of the presented embodiments.

In accordance with an aspect of the disclosure, a medical image processing method may include obtaining a plurality of actual medical images corresponding to a plurality of patients and including lesions; obtaining a first neural network, which is a trained deep neural network (DNN) based on the plurality of actual medical images for predicting a variation in a lesion over time, the lesion being included in a first medical image of the plurality of actual medical images, wherein the first medical image is obtained at a first time point; and generating, via the first neural network, a second medical image representing a state of the lesion at a second time point different from the first time point.

The second medical image may be an artificial medical image obtained by predicting a change state of the lesion included in the first medical image at the second time point different from the first time point.

The first neural network may predict at least one of a developing or changing form of the lesion included in each of the plurality of actual medical images over time, a possibility that an additional disease occurs due to the lesion, and a developing or changing form of the additional disease over time due to the lesion, and may output an artificial medical image including a result of the predicting as the second medical image.

The state of the lesion may include at least one of a generation time of the lesion, a developing or changing form of the lesion, a possibility that an additional disease occurs due to the lesion, and a developing or changing form of the additional disease due to the lesion.

The medical image processing method may further include training the first neural network, based on the second medical image, to adjust weighted values of a plurality of nodes that form the first neural network; and obtaining a second neural network including the adjusted weighted values.

The medical image processing method may further include analyzing a third medical image obtained by scanning an object of an examinee via the second neural network, and obtaining diagnosis information corresponding to the object of the examinee as a result of the analysis.

The diagnosis information may include at least one of a type of a disease having occurred in the object, characteristics of the disease, a possibility that the disease changes or develops over time, a type of an additional disease occurring due to the disease, characteristics of the additional disease, and a changing or developing state of the additional disease over time.

The medical image processing method may further include displaying a screen image including the second medical image.

The second medical image may be an X-ray image representing an object including the lesion.

The second medical image may be a lesion image representing the state of the lesion at the second time point different from the first time point.

In accordance with another aspect of the disclosure, a medical image processing apparatus may include a data obtainer configured to obtain a plurality of actual medical images corresponding to a plurality of patients and including lesions; and a controller configured to obtain a first neural network, which is a trained deep neural network (DNN) based on the plurality of actual medical images, for predicting a variation in a lesion over time. The lesion may be included in a first medical image of the plurality of actual medical images, and the first medical image may be obtained at a first time point. The controller may be further configured to generate, via the first neural network, a second medical image representing a state of the lesion at a second time point different from the first time point.

The second medical image may be an artificial medical image obtained by predicting a change state of the lesion included in the first medical image at the second time point different from the first time point.

The first neural network may predict at least one of a developing or changing form of the lesion included in each of the plurality of actual medical images over time, a possibility that an additional disease occurs due to the lesion, and a developing or changing form of the additional disease over time due to the lesion, and may output an artificial medical image including a result of the predicting as the second medical image.

The state of the lesion may include at least one of a generation time of the lesion, a developing or changing form of the lesion, a possibility that an additional disease occurs due to the lesion, characteristics of the additional disease, and a developing or changing form of the additional disease due to the lesion.

The controller may be further configured to train the first neural network, based on the second medical image, to adjust weighted values of a plurality of nodes that form the first neural network, and obtain a second neural network including the adjusted weighted values.

The controller may be further configured to analyze a third medical image obtained by scanning an object of an examinee via the second neural network, and obtain diagnosis information corresponding to the object of the examinee as a result of the analysis.

The diagnosis information may include at least one of a type of a disease having occurred in the object, characteristics of the disease, a possibility that the disease changes or develops over time, a type of an additional disease occurring due to the disease, characteristics of the additional disease, and a possibility that the additional disease changes or develops.

The second medical image may be at least one of an X-ray image representing an object including the lesion, and a lesion image representing the state of the lesion at the second time point different from the first time point.

The medical image processing apparatus may further include a display configured to display a screen image including the second medical image.

In accordance with another aspect of the disclosure, a non-transitory computer-readable recording medium may have recorded thereon instructions which, when executed by a processor, cause the processor to perform operations including: obtaining a plurality of actual medical images corresponding to a plurality of patients and including lesions; obtaining a first neural network, which is a trained deep neural network (DNN) based on the plurality of actual medical images, for predicting a variation in a lesion over time, the lesion being included in a first medical image of the plurality of actual medical images, wherein the first medical image is obtained at a first time point; and generating, via the first neural network, a second medical image representing a state of the lesion at a second time point different from the first time point.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is an external view and block diagram of a configuration of an X-ray apparatus according to an embodiment;
FIG. 2 is a block diagram of a medical image processing apparatus according to an embodiment;
FIG. 3 is a block diagram of a medical image processing apparatus according to an embodiment;
FIG. 4A is a block diagram of a deep neural network (DNN) processor included in a medical image processing apparatus according to an embodiment;
FIG. 4B is a block diagram of a data learner included in a DNN processor, according to an embodiment;
FIG. 4C is a block diagram of a data recognizer included in a DNN processor, according to an embodiment;
FIG. 5 is a diagram illustrating a learning operation according to an embodiment;
FIG. 6 is a view illustrating medical images that are used in a medical image processing apparatus according to an embodiment;
FIG. 7 is a view for describing generation of a second medical image according to an embodiment;
FIG. 8A is a view illustrating an example of second medical images generated according to an embodiment;
FIG. 8B is a view illustrating another example of a second medical image generated according to an embodiment;
FIG. 9 is a view for describing generation of a second medical image, according to an embodiment;
FIG. 10 is a diagram illustrating generation of diagnosis information, according to an embodiment;
FIG. 11 is a flow chart of a medical image processing method according to an embodiment; and
FIG. 12 is a flow chart of a medical image processing method according to another embodiment.

### DETAILED DESCRIPTION

Certain example embodiments are described in greater detail below with reference to the accompanying drawings.

In the following description, the same reference numerals are used for the same elements even when referring to different drawings. The matters defined in the description, such as detailed construction and elements, are provided to assist in a comprehensive understanding of example embodiments. Thus, it is apparent that example embodiments can be carried out without those specifically defined matters. Also, well-known functions or constructions are not described in detail since they would obscure example embodiments with unnecessary detail.

Terms such as "part," "portion," "module," "unit," "component," etc. as used herein denote those that may be embodied by software (e.g., code, instructions, programs, applications, firmware, etc.), hardware (e.g., circuits, or a combination of both software and hardware. According to example embodiments, a plurality of parts or portions may be embodied by a single unit or element, or a single part or portion may include a plurality of elements.

In the present specification, an image may include a medical image obtained by a magnetic resonance imaging (MRI) apparatus, a computed tomography (CT) apparatus, an ultrasound imaging apparatus, an X-ray apparatus, or another medical imaging apparatus.

Furthermore, in the present specification, an "object" may be a target to be imaged and may include a human, an animal, or a part of a human or animal. For example, the object may include a body part (e.g., an organ, tissue, etc.) or a phantom.

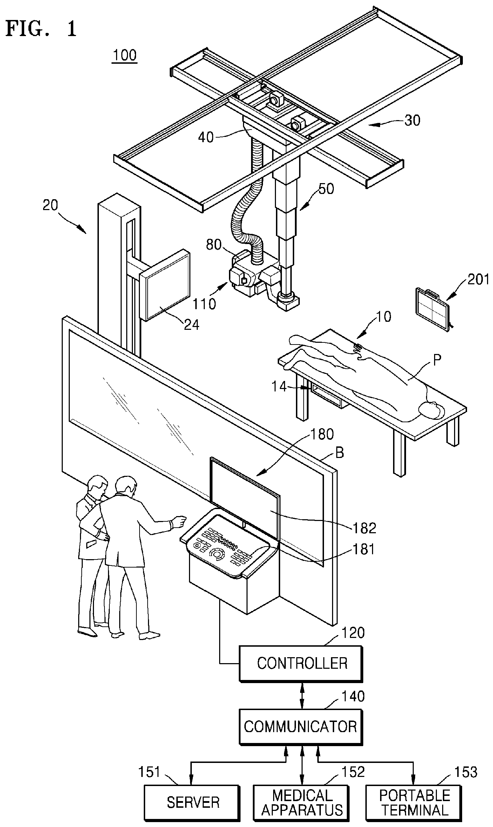

An X-ray apparatus capable of fast and conveniently obtaining a medical image, from among the above-described medical imaging apparatuses, will now be taken as an example, and will now be described in detail with reference to FIG. 1.

FIG. 1 is an external view and block diagram of a configuration of an X-ray apparatus 100 according to an embodiment.

In FIG. 1, it is assumed that the X-ray apparatus 100 is a fixed X-ray apparatus.

Referring to FIG. 1, the X-ray apparatus 100 includes an X-ray radiation device for generating and emitting X-rays, an X-ray detector 201 for detecting X-rays that are emitted by the X-ray radiation device 110 and transmitted through an object P, and a workstation 180 for receiving a command from a user and providing information to the user.

The X-ray apparatus 100 may further include a controller 120 for controlling the X-ray apparatus 100 according to the received command, and a communicator 140 for communicating with an external device.

All or some components of the controller 120 and the communicator 140 may be included in the workstation 180 or be physically separate from the workstation 180.

The X-ray radiation device 110 may include an X-ray source for generating X-rays and a collimator for adjusting a region irradiated with the X-rays generated by the X-ray source.

A guide rail 30 may be provided on a ceiling of an examination room in which the X-ray apparatus 100 is located, and the X-ray radiation device 110 may be coupled to a moving carriage 40 that is movable along the guide rail 30 such that the X-ray radiation device 110 may be moved to a position corresponding to the object P. The moving carriage 40 and the X-ray radiation device 110 may be connected to each other via a foldable post frame 50 such that a height of the X-ray radiation device 110 may be adjusted.

The workstation 180 may include an input device 181 for receiving a user command and a display 182 for displaying information.

The input device 181 may receive commands for controlling imaging protocols, imaging conditions, imaging timing, and locations of the X-ray radiation device 110.

The input device 181 may include a keyboard, a mouse, a touch screen, a microphone, a voice recognizer, etc.

The display 182 may display a screen for guiding a user's input, an X-ray image, a screen for displaying a state of the X-ray apparatus 100, and the like.

The controller 120 may control imaging conditions and imaging timing of the X-ray radiation device 110 according to a command input by the user and may generate a medical image based on image data received from an X-ray detector 201.

Furthermore, the controller 120 may control a position or orientation of the X-ray radiation device 110 or mounting units 14 and 24, each having the X-ray detector 201 mounted therein, according to imaging protocols and a position of the object P.

The controller 120 may include a memory configured to store programs for performing the operations of the X-ray apparatus 100 and a processor or a microprocessor configured to execute the stored programs.

The controller 120 may include a single processor or a plurality of processors or microprocessors. When the controller 120 includes the plurality of processors, the plurality of processors may be integrated onto a single chip or be physically separated from one another.

The X-ray apparatus 100 may be connected to external devices such as an external server 151, a medical apparatus 152, and/or a portable terminal 153 (e.g., a smart phone, a tablet personal computer (PC), or a wearable device) in order to transmit or receive data via the communicator 140.

The communicator 140 may include at least one component that enables communication with an external device. For example, the communicator 140 may include at least one of a local area communication module, a wired communication module, and a wireless communication module. The communication 140 may communicate via, for example, wireless local area network (WLAN), Wi-Fi, Bluetooth, the Internet, etc.

The communicator 140 may receive a control signal from an external device and transmit the received control signal to the controller 120 so that the controller 120 may control the X-ray apparatus 100 according to the received control signal.

In addition, by transmitting a control signal to an external device via the communicator 140, the controller 120 may control the external device according to the control signal.

For example, the external device may process data of the external device according to the control signal received from the controller 120 via the communicator 140

The communicator 140 may further include an internal communication module that enables communications between components of the X-ray apparatus 100.

A program for controlling the X-ray apparatus 100 may be installed on the external device and may include instructions for performing some or all of the operations of the controller 120.

The program may be preinstalled on the portable terminal 153, or a user of the portable terminal 153 may download the program from a server providing an application for installation.

The server that provides applications may include a recording medium where the program is stored.

Furthermore, the X-ray detector 201 may be implemented as a fixed X-ray detector that is fixedly mounted to a stand 20 or a table 10 or as a portable X-ray detector that may be detachably mounted in the mounting unit 14 or 24 or can be used at arbitrary positions.

The portable X-ray detector may be implemented as a wired or wireless detector according to a data transmission technique and a power supply method.

The X-ray detector 201 may or may not be a component of the X-ray apparatus 100.

If the X-ray detector 201 is not a component of the X-ray apparatus 100, the X-ray detector 201 may be registered by the user for use with the X-ray apparatus 100.

Furthermore, in both cases, the X-ray detector 201 may be connected to the controller 120 via the communicator 140 to receive a control signal from or transmit image data to the controller 120.

A sub-user interface 80 that provides information to a user and receives a command from the user may be provided on one side of the X-ray radiation device 110. The sub-user interface 80 may also perform some or all of the functions performed by the input device 181 and the display 182 of the workstation 180.

When all or some components of the controller 120 and the communicator 140 are separate from the workstation 180, they may be included in the sub-user interface 80 provided on the X-ray radiation device 110.

Although FIG. 1 shows a fixed X-ray apparatus connected to the ceiling of the examination room, examples of the X-ray apparatus 100 may include a C-arm type X-ray apparatus, a mobile X-ray apparatus, and other X-ray apparatuses having various structures that will be apparent to those of ordinary skill in the art.

To improve ease of reading of a medical image or diagnosis using the medical image, a medical image processing apparatus may analyze a medical image obtained by a medical imaging apparatus and may utilize a result of the analysis. The medical image may be any image that represents the inside of an object of a patient. The medical image may be referred to as not only an image that visually represents an object, but also data that is obtained to generate an image.

A medical image obtained by directly scanning an object of a patient by using a medical imaging apparatus will now be referred to as an actual medical image, and a medical image obtained without directly imaging an object of a patient by using a medical imaging apparatus will now be referred to as an artificial medical image.

The medical image processing apparatus may refer to any electronic apparatus capable of obtaining certain information by using a medical image, or obtaining diagnosis information by analyzing a medical image, or processing, generating, correcting, updating, or displaying all images or information for use in diagnosis, based on a medical image.

In detail, the medical image processing apparatus may analyze a medical image obtained by a medical imaging apparatus by using a computer, according to image processing technology, such as a computer aided detection (CAD) system or machine learning, and may use a result of the analysis.

A medical image processing method according to an embodiment for obtaining at least one medical image from which a variation in a detected lesion over time may be predicted, when a lesion is included in or detected from a medical image captured by a medical imaging apparatus, such as the X-ray apparatus 100 of FIG. 1, at a certain time point, and a medical image processing apparatus performing the medical image processing method will now be described in detail with reference to the accompanying drawings.

The medical image processing apparatus according to an embodiment may include any electronic apparatus capable of obtaining additional information for use in diagnosis by processing a medical image. The processing of the medical image may include all of the operations of analyzing a medical image, processing the medical image, and generating, analyzing, and displaying data produced as a result of analyzing the medical image.

The medical image processing apparatus according to an embodiment may be realized in various types. For example, the medical image processing apparatus according to an embodiment may be mounted on a workstation (for example, the workstation 180 of the X-ray apparatus 100) or a console of a medical imaging apparatus, for example, the X-ray apparatus 100 of FIG. 1, a CT apparatus, an MRI system, or an ultrasound diagnosis apparatus.

As another example, the medical image processing apparatus according to an embodiment may be mounted on a special apparatus or server independent from a medical imaging apparatus, for example, the X-ray apparatus 100 of FIG. 1, a CT apparatus, an MRI system, or an ultrasound diagnosis apparatus. The special apparatus or server independent from a medical imaging apparatus may be referred to as an external apparatus. For example, the external apparatus may be the server 151, the medical apparatus 152, or the portable terminal 153 of FIG. 1, and may receive an actual medical image from the medical imaging apparatus via a wired and wireless communication network. For example, the medical image processing apparatus according to an embodiment may be mounted on a workstation for analysis, an external medical apparatus, a Picture Archiving Communications System (PACS) server, a PACS viewer, an external medical server, or a hospital server.

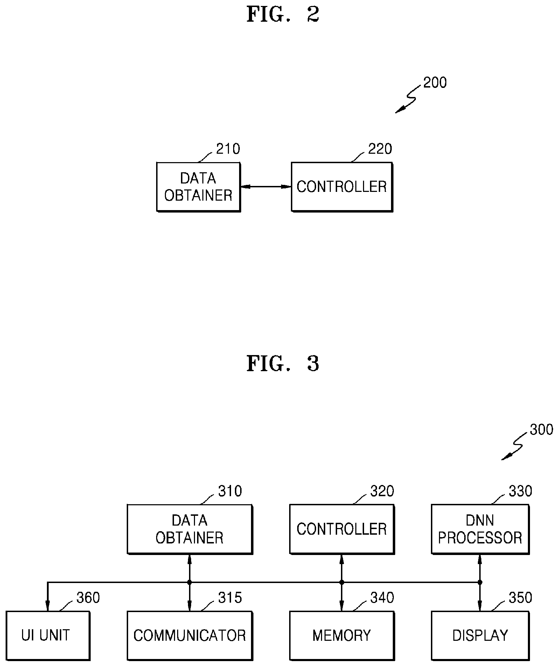

FIG. 2 is a block diagram of a medical image processing apparatus 200 according to an embodiment.

Referring to FIG. 2, the medical image processing apparatus 200 may include a data obtainer 210 and a controller 220. Various modules, units, and components illustrated in FIG. 2 and other figures may be implemented with software, hardware, or a combination of both.

The data obtainer 210 obtains a plurality of actual medical images corresponding to a plurality of patients and including lesions. The plurality of actual medical images are medical images including lesions, and are pieces of data that are used to train a DNN. Accordingly, the plurality of actual medical images include lesions having various shapes, states, and progresses, and may be images obtained by scanning patients having various ages, genders, and family histories.

A lesion may refer to any abnormal body part other than a body part including at least one of a cell, tissue, organ, and component material of a healthy body. Accordingly, the lesion may include all of a body part immediately before a disease is present, and a body part having a disease.

The data obtainer 210 may obtain a plurality of actual medical images according to various methods. For example, when the medical image processing apparatus 200 is formed within a medical imaging apparatus (for example, the X-ray apparatus 100 of FIG. 1), the medical image processing apparatus 200 may autonomously obtain a medical image by performing medical imaging. As another example, when the medical image processing apparatus 200 and a medical imaging apparatus are formed as independent apparatuses, the medical image processing apparatus 200 may receive a medical image from the medical imaging apparatus via a wired and wireless communication network. In this case, the data obtainer 210 may include a communicator (for example, a communicator 315 to be described later with reference to FIG. 3) and may receive a plurality of actual medical images via the communicator.

The controller 220 trains a DNN, based on the plurality of actual medical images obtained by the data obtainer 210. The controller 220 obtains a first neural network for predicting a variation in a lesion over time, as a result of the training. The first neural network is a trained DNN for predicting a variation in a cultured lesion over time. Accordingly, the first neural network works for generating an artificially cultured lesion. The controller 220 obtains at least one second medical image visually representing a state of a lesion included in a first medical image included in the plurality of actual medical images at at least one time point different from a first time point, which is a time point when the first medical image is obtained, via the first neural network. For convenience of explanation, the first medical image has been recited as a singular form, but the first medical image may include a plurality of different medical images. In other words, the controller 220 may generate at least one second medical image respectively corresponding to at least one first medical image included in the plurality of actual medical images via the first neural network.

The actual medical images and the second medical image represent the inside of an object, and thus may include an X-ray image, a CT image, an MR image, and an ultrasound image. The plurality of actual medical images, from which various shapes of lesions may be ascertained, may be images obtained by performing medical imaging on a plurality of patients having lesions having different progresses and shapes.

The DNN performs an operation for inferring and prediction according to the AI technology. In detail, a DNN operation may include a convolution neural network (CNN) operation. In other words, the controller 220 may implement a data recognition model via the above-illustrated neural network, and may train the implemented data recognition model by using training data. The controller 220 may analyze or classify a medical image, which is input data, by using the trained data recognition model, and thus analyze and classify what abnormality has occurred within an object image from the medical image.

In detail, the first neural network may perform an operation for predicting at least one of seriousness of a certain lesion included in each of the plurality of actual medical images, the possibility that the certain lesion corresponds to a certain disease, a developing or changing form of the certain lesion over time, the possibility that an additional disease occurs due to the certain lesion, and a developing or changing form of the additional disease over time due to the certain lesion, and outputting an artificial medical image including a result of the prediction.

The state of a lesion represented by a second medical image may include at least one of a generation time (e.g., a time of occurrence) of the lesion, a developing or changing form of the lesion, the possibility that an additional disease occurs due to the lesion, and a developing or changing form of the additional disease due to the lesion.

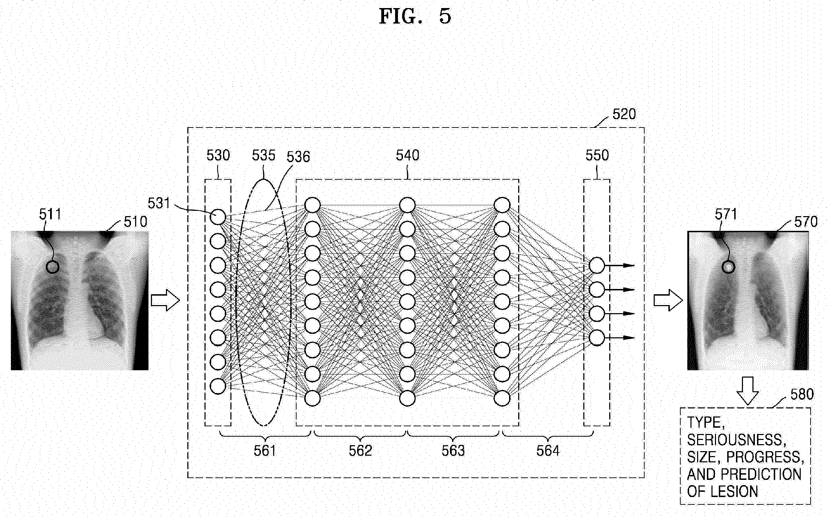

A case where a currently-present lesion, which is a lesion included in a first medical image, is stage 0 lung cancer will now be illustrated. In this case, a second medical image is at least one image representing a change or development aspect of the lesion, which is a stage 0 lung cancer, over time, a changed state of the lesion over time, and spreading or non-spreading of the lesion over time or a spreading degree (or state) of the lesion, and thus may be an artificial medical image obtained by performing an operation using a DNN 520 of FIG. 5.

When the possibility that the stage 0 lung cancer lesion, which is the currently-present lesion, is spread to another organ at a subsequent time point is high, the second medical image may be an image representing information about metastatic cancer, for example, metastatic brain cancer, that may occur at a certain subsequent time point, for example, three years after a current time point, due to the stage 0 lung cancer, which is the cause of the currently-present lesion. In this case, the second medical image may include an image or data that represents a location, shape, and characteristics of the metastatic brain cancer that is determined to be highly likely to occur three years after the current time point.

The controller 220 may use machine learning technology other than AI-based machine learning, in order to predict a variation in the lesion over time by training the plurality of actual medical images. In detail, machine learning is an operation technique for detecting a lesion included in a medical image via a computer operation and analyzing characteristics of the detected lesion to thereby predict a variation in the lesion at a subsequent time point, and may be a CAD operation, data-based statistical learning, or the like.

Accordingly, the controller 220 may train the plurality of actual medical images to predict a variation in the lesion over time, via machine learning, such as a CAD operation or data-based statistical learning. The controller 220 may obtain at least one second medical image representing a state of a lesion included in a first medical image included in the plurality of actual medical images at at least one time point different from a first time point, which is a time point when the first medical image is obtained, by using a result of the prediction. Each of the at least one second medical image may be an artificial medical image obtained by predicting a change state of the lesion included in the first medical image at each of the at least one time point different from the first time point. In other words, the second medical image is not an image obtained by imaging a lesion by scanning an object, but is a medical image artificially generated as a result of an operation via the first neural network.

The controller 220 may include a memory, for example, read-only memory (ROM) or random access memory (RAM), and at least one processor that executes commands for performing the above-described operations. The at least one processor included in the controller 220 may operate to execute the commands for performing the above-described operations.

The medical image processing apparatus according to an embodiment may cure a lesion by using AI technology, based on pieces of lesion information of a patient checked from a medical image. In other words, the medical image processing apparatus according to an embodiment cures a lesion by using AI technology in order to ascertain a state of the lesion at at least one time point different from a time point when an actual medical image has been obtained. An image including the AI cured lesion may be the aforementioned second medical image. Accordingly, in addition to the state of the lesion at the time point when the actual medical image has been obtained, a user, such as a doctor, is able to easily predict or ascertain a development aspect (or state) of the lesion at a subsequent time point via the second medical image.

The controller 220 may train the first neural network, based on the at least one second medical image, to adjust weighted values of a plurality of nodes that form the first neural network, and may obtain a second neural network including the adjusted weighted values. In other words, the second neural network may be obtained by correcting or updating the first neural network.

The controller 220 may analyze a third medical image obtained by scanning an object of an examinee via the second neural network, and may obtain diagnosis information corresponding to the object of the examinee as a result of the analysis. The diagnosis information may include at least one of the type of disease having occurred in the object, the characteristics of the disease, the possibility that the disease changes or develops over time, the type of additional disease that may occur due to the previous disease, the characteristics of the additional disease, and the possibility that the additional disease changes or develops over time.

A DNN, such as the first and second neural networks, will be described in detail later with reference to FIG. 5.

FIG. 3 is a block diagram of a medical image processing apparatus 300 according to an embodiment. A data obtainer 310 and a controller 320 of the medical image processing apparatus 300 of FIG. 3 may be respectively the same as the data obtainer 210 and the controller 220 of the medical image processing apparatus 200 of FIG. 2, and thus repeated descriptions thereof will be omitted.

Referring to FIG. 3, the medical image processing apparatus 300 may further include at least one of a communicator 315, a DNN processor 330, a memory 340, a display 350, and a user interface (UI) unit 360, compared with the medical image processing apparatus 200 of FIG. 2.

In the medical image processing apparatus 200 of FIG. 2, the controller 220 performs an operation via a DNN, for example, learning. However, in the medical image processing apparatus 300 of FIG. 3, a dedicated processor may perform an operation via a DNN. In detail, at least one processor that performs an operation via a DNN may be referred to as a DNN processor 330.

The DNN processor 330 may perform an operation based on a neural network. In detail, a DNN operation may include a CNN operation.

In detail, the DNN processor 330 trains a DNN, based on a plurality of actual medical images obtained by the data obtainer 310. The DNN processor 330 obtains a first neural network for predicting a variation in a lesion over time, as a result of the training. The DNN processor 330 obtains at least one second medical image representing a state of a lesion included in a first medical image included in the plurality of actual medical images at at least one time point different from a first time point, which is a time point when the first medical image has been obtained, via the first neural network.

The at least one time point different from the first time point may be set by a user, the controller 320, or the DNN processor 330.

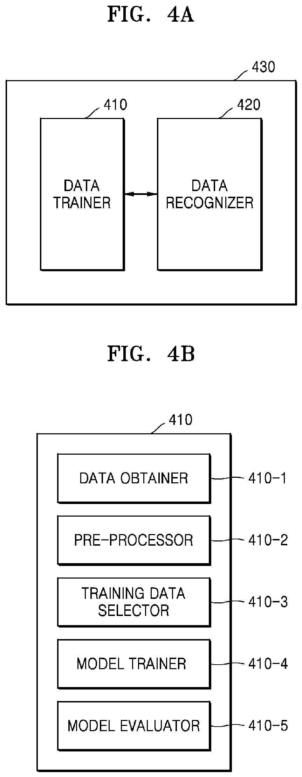

The DNN processor 330 may be included to be distinguished from the controller 320, as shown in FIG. 3. Alternatively, the DNN processor 330 may be at least one of one or more processors included in the controller 320. In other words, the DNN processor 330 may be included in the controller 320. A detailed structure of the DNN processor 330 will be described in detail later with reference to FIGS. 4A through 4C. A DNN operation that is performed by the controller 220 or 320 and/or the DNN processor 330 will be described in detail later with reference to FIGS. 5 through 10.

The communicator 315 may transmit and receive data to and from an electronic apparatus via a wired-wireless communication network. In detail, the communicator 315 may perform data transmission and data reception under the control of the controller 320. The communicator 315 may correspond to the communicator 140 of FIG. 1. The electronic apparatus that is connected to the communicator 315 via a wired and wireless communication network may be the server 151, the medical apparatus 152, or the portable terminal 153 of FIG. 1. The electronic apparatus (not shown) may be a medical imaging apparatus formed independently from the medical image processing apparatus 300, for example, may be the X-ray apparatus 100 of FIG. 1.

In detail, when the external electronic apparatus is a medical imaging apparatus, the communicator 315 may receive an actual medical image obtained by the medical imaging apparatus. The communicator 315 may transmit the at least one second medical image to the external electronic apparatus. The communicator 315 may transmit at least one of information, data, and an image generated by the controller 320 or the DNN processor 330 to the external electronic apparatus.

The memory 340 may include at least one program necessary for the medical image processing apparatus 300 to operate, or at least one instruction necessary for the at least one program to be executed. The memory 340 may also include one or more processors for performing the above-described operations.

The memory 340 may store at least one of a medical image, information associated with the medical image, information about a patient, and information about an examinee. The memory 340 may store at least one of the information, the data, and the image generated by the controller 320 or the DNN processor 330. The memory 340 may store at least one of an image, data, and information received from the external electronic apparatus.

The display 350 may display a medical image, a UI screen image, user information, image processing information, and the like. In detail, the display 350 may display a UI screen image generated under the control of the controller 320. The UI screen image may include the medical image, the information associated with the medical image, and/or the information generated by the controller 320 or the DNN processor 330.

According to an embodiment, the display 350 may display a UI screen image including at least one of an actual medical image, a second medical image, and diagnosis information.

The UI unit 360 may receive certain data or a certain command from a user. The UI unit 360 may correspond to at least one of the sub-user interface 80 and the input device 181 of FIG. 1. The UI unit 360 may be implemented using a touch screen integrally formed with the display 350. As another example, the UI unit 360 may include a user input device, such as a pointer, a mouse, or a keyboard.

FIG. 4A is a block diagram of a DNN processor 430 included in a medical image processing apparatus according to an embodiment. The DNN processor 430 of FIG. 4A is the same as the DNN processor 330 of FIG. 3, and thus a repeated description thereof will be omitted.

Referring to FIG. 4A, the DNN processor 430 may include a data trainer 410 and a data recognizer 420.

The data trainer 410 may train a criterion for performing an operation via the above-described DNN. In detail, the data trainer 410 may train a criterion regarding what data is used to predict a variation in a lesion over time and how to determine a situation by using data. The data trainer 410 may obtain data for use in training and may apply the obtained data to a data recognition model which will be described later, thereby training the criterion for situation determination. The data that the data trainer 410 uses during training may be a plurality of actual medical images obtained by the data obtainer 310.

The data recognizer 420 may determine a situation based on data. The data recognizer 420 may recognize a situation from certain data, by using the trained data recognition model, for example, the first neural network.

In detail, the data recognizer 420 may obtain certain data according to a criterion previously set due to training, and use a data recognition model by using the obtained data as an input value, thereby determining a situation based on the certain data. A result value output by the data recognition model by using the obtained data as an input value may be used to update the data recognition model.

According to an embodiment, the data recognition model established by the data recognizer 420, for example, the first neural network generated by training the DNN 520 or the second neural network generated by training the first neural network, may be modeled to infer (e.g., predict) change characteristics of a lesion included in each of the plurality of actual medical images over time by training the plurality of actual medical images. In other words, the data recognition model established by the data recognizer 420 infers a development or change form of a certain lesion over time, characteristics of the developing or changing lesion, and/or a shape or characteristics of an additional disease that occurs due to a development or change of the certain lesion.

In detail, the data recognizer 420 obtains at least one second medical image representing a state of a lesion included in a first medical image included in the plurality of actual medical images at at least one time point different from a first time point, which is a time point when the first medical image has been obtained, via the first neural network. Data that is input to the first neural network, which is a data recognition model, may be the first medical image included in the plurality of actual medical images, and may be the at least one second medical image that is output via the first neural network.

At least one of the data trainer 410 and the data recognizer 420 may be manufactured in the form of at least one hardware chip and may be mounted on an electronic apparatus. For example, at least one of the data trainer 410 and the data recognizer 420 may be manufactured in the form of a dedicated hardware chip for AI, or may be manufactured as a portion of an existing general-purpose processor (for example, a central processing unit (CPU) or an application processor (AP)) or a processor dedicated to graphics (for example, a graphics processing unit (GPU)) included in the controller 320 or separate from the controller 320, and thus may be mounted on any of the aforementioned various electronic apparatuses.

The data trainer 410 and the data recognizer 420 may be both mounted on the medical image processing apparatus 300, which is a single electronic apparatus, or may be respectively mounted on independent electronic apparatuses. For example, one of the data trainer 410 and the data recognizer 420 may be included in the medical image processing apparatus 300, and the other may be included in a server. The data trainer 410 and the data recognizer 420 may be connected to each other by wire or wirelessly, and thus model information established by the data trainer 410 may be provided to the data recognizer 420 and data input to the data recognizer 420 may be provided as additional training data to the data trainer 410.

At least one of the data trainer 410 and the data recognizer 420 may be implemented as a software module. When at least one of the data trainer 410 and the data recognizer 420 is implemented using a software module (or a program module including instructions), the software module may be stored in non-transitory computer readable media. In this case, the at least one software module may be provided by an operating system (OS) or by a certain application. Alternatively, some of the at least one software module may be provided by an OS and the others may be provided by a certain application.

FIG. 4B is a block diagram of the data trainer 410 included in the DNN processor 430, according to an embodiment.

Referring to FIG. 4B, the data trainer 410 according to an embodiment may include a pre-processor 410-2, a training data selector 410-3, a model trainer 410-4, and a model evaluator 410-5.

The pre-processor 410-2 may pre-process obtained data such that the obtained data may be used in training for situation determination. According to an embodiment, the pre-processor 410-2 may process the obtained data in a preset format such that the model trainer 410-4, which will be described later, may use a plurality of actual medical images, which are the obtained data for use in training for situation determination.

The training data selector 410-3 may select data necessary for training from among the pre-processed data. The selected data may be provided to the model trainer 410-4. The training data selector 410-3 may select the data necessary for training from among the pre-processed data, according to the preset criterion for situation determination. The training data selector 410-3 may select data according to a criterion previously set due to training by the model trainer 410-4.

The model trainer 410-4 may train a criterion regarding how to determine a situation, based on the training data. The model trainer 410-4 may train a criterion regarding which training data is to be used for situation determination.

According to an embodiment, the model trainer 410-4 may train the plurality of actual medical images and may train a criterion necessary for predicting a variation in a lesion, based on trained data. In detail, the model trainer 410-4 may classify lesions respectively included in the plurality of actual medical images according to the ages of patients, the types, tissue characteristics, or progresses (or progress stages or clinical stages) of lesions of the patients, and body parts of the patients having the lesions, and may train the characteristics of the classified lesions to thereby train a criterion necessary for predicting a progress, aspect, and characteristics of each of the classified lesions over time.

The model trainer 410-4 may train a data recognition model for use in situation determination, by using the training data. In this case, the data recognition model may be a previously established model. For example, the data recognition model may be a model previously established by receiving basic training data (for example, a sample medical image).

The data recognition model may be established in consideration of, for example, an application field of a recognition model, a purpose of learning, or computer performance of a device. The data recognition model may be, for example, a model based on a neural network. For example, a model, such as a DNN, a recurrent neural network (RNN), or a bidirectional recurrent DNN (BRDNN), may be used as the data recognition model, but embodiments are not limited thereto.

When the data recognition model is trained, the model trainer 410-4 may store the trained data recognition model. In this case, the model trainer 410-4 may store the trained data recognition model in a memory of an electronic apparatus including the data recognizer 420. Alternatively, the model trainer 410-4 may store the trained data recognition model in a memory of a server that is connected with the electronic apparatus via a wired or wireless network.

In this case, the memory that stores the first neural network, which is the trained data recognition model, may also store, for example, a command or data related with at least one other component of the electronic apparatus. The memory may also store software and/or a program. The program may include, for example, a kernel, middleware, an application programming interface (API), and/or an application program (or an application).

The model evaluator 410-5 may input evaluation data to the data recognition model. When a recognition result that is output from the evaluation data does not satisfy predetermined accuracy or predetermined reliability, which is a predetermined criterion, the model evaluator 410-5 may enable the model trainer 410-4 to perform training again. In this case, the evaluation data may be preset data for evaluating the data recognition model.

For example, when the number or percentage of pieces of evaluation data, which provide inaccurate recognition results from among recognition results of the trained data recognition model with respect to the evaluation data, exceeds a preset threshold, the model evaluator 410-5 may evaluate or determine that the recognition result does not satisfy the predetermined criterion. For example, when the predetermined criterion is defined as 2% and the trained data recognition model outputs wrong recognition results for more than 20 pieces of evaluation data from among a total of 1000 pieces of evaluation data, the model evaluator 410-5 may evaluate that the trained data recognition model is not appropriate.

When there are a plurality of trained data recognition models, the model evaluator 410-5 may evaluate whether each of the plurality of trained data recognition models satisfies the predetermined criterion, and may determine, as a final data recognition model, a data recognition model that satisfies the predetermined criterion.

At least one of the pre-processor 410-2, the training data selector 410-3, the model trainer 410-4, and the model evaluator 410-5 within the data trainer 410 may be manufactured in the form of at least one hardware chip and may be mounted on an electronic apparatus.

The pre-processor 410-2, the training data selector 410-3, the model trainer 410-4, and the model evaluator 410-5 may be all mounted on a single electronic apparatus, or may be respectively mounted on independent electronic apparatuses. For example, some of the pre-processor 410-2, the training data selector 410-3, the model trainer 410-4, and the model evaluator 410-5 may be included in an electronic apparatus, and the others may be included in a server.

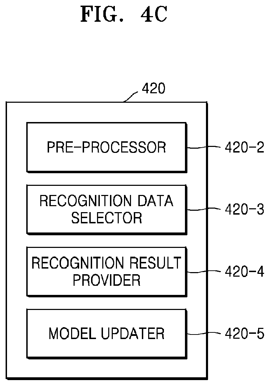

FIG. 4C is a block diagram of the data recognizer 420 included in the DNN processor 430, according to an embodiment.

Referring to FIG. 4C, the data recognizer 420 according to an embodiment may include a pre-processor 420-2, a recognition data selector 420-3, a recognition result provider 420-4, and a model updater 420-5.

And, the data recognizer 420 may further include a data obtainer. The data obtainer may obtain data necessary for situation determination, and the pre-processor 420-2 may pre-process the obtained data such that the obtained data may be used for situation determination. The pre-processor 420-2 may process the obtained data in a preset format such that the recognition result provider 420-4, which will be described later, may use the obtained data for situation determination.

The recognition data selector 420-3 may select data necessary for situation determination from among the pre-processed data. The selected data may be provided to the recognition result provider 420-4. The recognition data selector 420-3 may select some or all of the pre-processed data, according to the preset criterion for situation determination. The recognition data selector 420-3 may select data according to the criterion previously set due to the training by the model trainer 410-4.

The recognition result provider 420-4 may determine a situation by applying the selected data to the data recognition model. The recognition result provider 420-4 may provide a recognition result that conforms to a data recognition purpose.

According to an embodiment, the data obtainer may receive, for example, the first medical image of the plurality of actual medical images input to the first neural network, which is the data recognition model. The pre-processor 420-2 may pre-process the received first medical image. Then, the recognition data selector 420-3 may select the pre-processed first medical image. The recognition result provider 420-4 may generate and provide the at least one second medical image representing a state of the lesion included in the first medical image at at least one time point different from the first time point, which is a time point when the first medical image has been obtained.

For example, when the first medical image is an actual medical image obtained at the first time point, the first medical image represents the state of a lesion at the first time point. Because the first neural network is a data recognition model that predicts a variation in a lesion over time, the recognition result provider 420-4 may generate two second medical images representing states of lesions corresponding to two time points different from the first time point, which is a time point when the actual medical image has been obtained. For example, the second medical images may respectively correspond to time points at one month after the first time point and three months after the first time point.

The model updater 420-5 may enable the data recognition model to be updated, based on an evaluation of a recognition result provided by the recognition result provider 420-4. For example, the model updater 420-5 may enable the model trainer 410-4 to update the data recognition model, by providing the recognition result provided by the recognition result provider 420-4 to the model trainer 410-4.

According to an embodiment, every time an actual medical image is additionally obtained, the model updater 420-5 may enable the first neural network to be updated, by training the first neural network, which is a data recognition model. The model updater 420-5 may obtain a second neural network by correcting or updating the first neural network by training the first neural network by using the at least one second medical image obtained via an operation based on the first neural network.

At least one of the data obtainer 420-1, the pre-processor 420-2, the recognition data selector 420-3, the recognition result provider 420-4, and the model updater 420-5 within the data recognizer 420 may be manufactured in the form of at least one hardware chip and may be mounted on an electronic apparatus. The data obtainer 420-1, the pre-processor 420-2, the recognition data selector 420-3, the recognition result provider 420-4, and the model updater 420-5 may be all mounted on a single electronic apparatus, or may be respectively mounted on independent electronic apparatuses. For example, some of the data obtainer 420-1, the pre-processor 420-2, the recognition data selector 420-3, the recognition result provider 420-4, and the model updater 420-5 may be included in an electronic apparatus, and the others may be included in a server.

An operation performed via a DNN will now be described in more detail with reference to FIGS. 5 through 10. The operation performed via a DNN will be described by referring to the medical image processing apparatus 300 of FIG. 3.

FIG. 5 is a diagram illustrating a learning operation according to an embodiment.

The medical image processing apparatus 300 according to an embodiment performs an operation based on a DNN. In detail, the medical image processing apparatus 200 or 300 may train a DNN via the controller 220 or 320 or the DNN processor 330. The medical image processing apparatus 200 or 300 may perform an inferring operation based on the trained DNN. A case where the DNN processor 330 performs an operation based on a DNN will now be illustrated and described.

Referring to FIG. 5, the medical image processing apparatus 300 may train a DNN, based on the plurality of actual medical images, to thereby obtain the first neural network, which is the DNN 520 for predicting a variation of a lesion over time. Because the first neural network is a DNN trained based on the plurality of actual medical images, a neural network illustrated in block 520 may be referred to as a DNN, and may also be referred to as a first neural network for generating a cultured lesion.

Referring to FIG. 5, the DNN processor 330 may perform an operation via the DNN 520, which includes an input layer, a hidden layer, and an output layer. The hidden layer may include a plurality of layers, for example, a first hidden layer, a second hidden layer, and a third hidden layer.

Referring to FIG. 5, the DNN 520 includes an input layer 530, a hidden layer 540, and an output layer 550.

The DNN 520 is trained based on the plurality of actual medical images to thereby establish the first neural network for predicting a variation of a lesion over time.

In detail, the DNN 520 may analyze information included in the plurality of actual medical images, which is input data, to analyze lesions respectively included in the plurality of actual medical images, and thereby obtain information indicating the characteristics of the lesions. The DNN 520 may obtain a first neural network for predicting variations in the lesions over time, by training the obtained information.

For example, when the input data is an X-ray image 510, the DNN 520 may output, as output data, result data obtained by analyzing an object image included in the X-ray image 510. The DNN 520 trains the plurality of actual medical images, but FIG. 5 illustrates a case where the DNN 520 trains the X-ray image 510, which is one of the plurality of actual medical images.

The plurality of layers that form the DNN 520 may include a plurality of nodes 531 that receive data. As shown in FIG. 5, two adjacent layers are connected to each other via a plurality of edges 536. Because the nodes have weighted values, respectively, the DNN 520 may obtain output data, based on a value obtained by performing an arithmetic operation (e.g., multiplication) with respect to an input signal and each of the weighted values.

Referring to FIG. 5, the input layer 530 receives the X-ray image 510 obtained by scanning a chest, which is an object. The X-ray image 510 may be an image obtained by scanning an object having a lesion 511 on his or her right chest at a first time point.

Referring to FIG. 5, the DNN 520 may include a first layer 561 formed between the input layer 530 and the first hidden layer, a second layer 562 formed between the first hidden layer and the second hidden layer, a third layer 563 formed between the second hidden layer and the third hidden layer, and a fourth layer 564 formed between the third hidden layer and the output layer 550.

The plurality of nodes included in the input layer 530 of the DNN 520 receive a plurality of pieces of data corresponding to the X-ray image 510. The plurality of pieces of data may be a plurality of partial images generated by performing filter processing of splitting the X-ray image 510.

Via operations in the plurality of layers included in the hidden layer 540, the output layer 550 may output pieces of output data 570 and 580 corresponding to the X-ray image 510. In the shown illustration, because the DNN 520 performs an operation to obtain a result of analyzing the characteristics of a lesion included in the input X-ray image 510, the output layer 550 may output an image 570 displaying a lesion 571 detected from the input X-ray image 510 and/or data 580 obtained by analyzing the detected lesion 571. The data 580 is information indicating the characteristics of the detected lesion 571, and may include the type, severity, progress, size, and location of the lesion 571. The data 580 may be classified by patients and obtained. For example, the data 580 may be classified by the genders, ages, and family histories of patients and obtained.

To increase the accuracy of output data output via the DNN 520, learning may be performed in a direction from the output layer 550 to the input layer 530, and the weighted values may be adjusted such that the accuracy of output data increases. Accordingly, the DNN 520 may perform deep learning by using a plurality of different actual medical images to adjust the respective weighted values of the nodes toward detecting the characteristics of the lesion included in the X-ray image.

Then, the DNN 520 may be updated to the first neural network capable of automatically performing an operation for predicting a variation of a lesion over time, based on lesion characteristics obtained by training the plurality of actual medical images.

Accordingly, the DNN 520 (e.g., the first neural network) may receive a first medical image included in the plurality of actual medical images and obtained at a first time point and may output at least one second medical image representing a changed state of a lesion included in the first medical image at at least one time point different from the first time point, which is a result of predicting a variation in the lesion. The DNN 520 may also generate a plurality of second medical images respectively corresponding to the plurality of actual medical images.

The DNN 520 (e.g., the first neural network) may train the generated plurality of second medical images to thereby train the DNN 520 toward increasing the accuracy of output data. In other words, the DNN 520 may establish a second neural network, which is the updated DNN 520, by adjusting or correcting the weighted values corresponding to the plurality of nodes that form the DNN 520, via training using each of the plurality of second medical images, which are artificial medical images.

Various pieces of training data are needed to increase the accuracy of an inferring operation that is performed by the DNN 520. In other words, the accuracy of a result of inferring new input data may be increased by training the DNN 520 by training various pieces of training data.

As described above, as actual medical images obtained from actual patients are trained, via the DNN 520, a plurality of second medical images representing states of lesions at various time points, which are different from time points when the actual medical images have been obtained, may be obtained. The diversity of training data may be increased by re-training the DNN 520 by using the obtained plurality of second medical images. For example, when there are 1000 actual medical images and four second medical images representing progress states of lesions at four different time points are obtained from each of the actual medical images, a total of 4000 different artificial medical images may be obtained. When the DNN 520 is trained using the 4000 different artificial medical images, the accuracy of an operation based on the DNN 520 may increase.

Accordingly, a more accurate DNN 520 may be established by diversifying the training data and increasing the amount of the training data by overcoming the limitation of a medical image database (DB).

Moreover, by referring to at least one second medical image, a user, such as a doctor, may easily ascertain a variation in an actually scanned lesion over time. In other words, the user, such as a doctor, may easily ascertain and predict occurrence of the lesion, development thereof, and/or the possibility that the lesion changes (e.g., morphs).

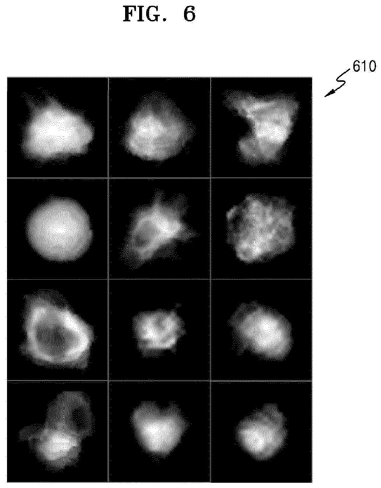

FIG. 6 is a view illustrating medical images that are used in a medical image processing apparatus according to an embodiment.

FIG. 5 illustrates a case where actual medical images for use in training of the DNN 520 are medical images representing the entire object.

The actual medical images for use in training of the DNN 520 may be a plurality of lesion images 610, as shown in FIG. 6. In detail, each of the plurality of lesion images 610 is an image generated by extracting only a portion having a lesion from an actual medical image.

When the DNN 520 trains the plurality of lesion images 610, the DNN 520 may immediately analyze and ascertain the characteristics of each of the plurality of lesion images 610 without extraction operations for obtaining the plurality of lesion images 610.

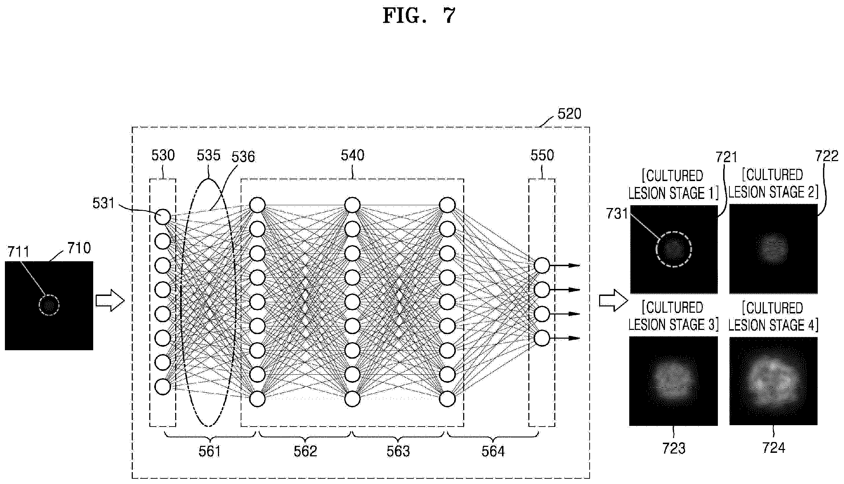

FIG. 7 is a view for describing generation of a second medical image according to an embodiment.

Referring to FIG. 7, the medical image processing apparatus 300 according to an embodiment may receive a lesion image 710 (as an actual medical image, and may obtain at least one artificial lesion image, namely, four artificial lesion images 721, 722, 723, and 724, which are at least one second medical image corresponding to the lesion image 710. Referring to FIG. 7, the lesion image 710 may correspond to one of a plurality of lesion images 610.

In detail, the medical image processing apparatus 300 may input a lesion image 710, which is an actual medical image obtained by imaging a lesion at a first time point, to the DNN 520, and may obtain a plurality of artificial lesion images 721, 722, 723, and 724 corresponding to a plurality of time points different from the first time point, via an operation based on the DNN 520. For example, when the lesion image 710 is an initial lesion image obtained by scanning an object at a current time point, the medical image processing apparatus 300 may obtain the four artificial lesion images 721, 722, 723, and 724 respectively including artificially cultured lesion and representing progress states of the lesion at subsequent time points. In detail, the artificial medical images 721, 722, 723, and 724 output by the DNN 520 may include a cultured lesion stage 1 image 721 representing a case where a lesion 711 included in the lesion image 710 proceeds to stage 1, a cultured lesion stage 2 image 722 representing a case where the lesion 711 proceeds to stage 2, a cultured lesion stage 3 image 723 representing a case where the lesion 711 proceeds to stage 3, and a cultured lesion stage 4 image 724 representing a case where the lesion 711 proceeds to stage 4.

Accordingly, the medical processing imaging apparatus 300 according to an embodiment may easily and accurately ascertain and predict changed states of the lesion 711 at time points subsequent to a current time point when the lesion 711 has been detected, via the plurality of artificial lesion images 721, 722, 723, and 724 output by the DNN 520.

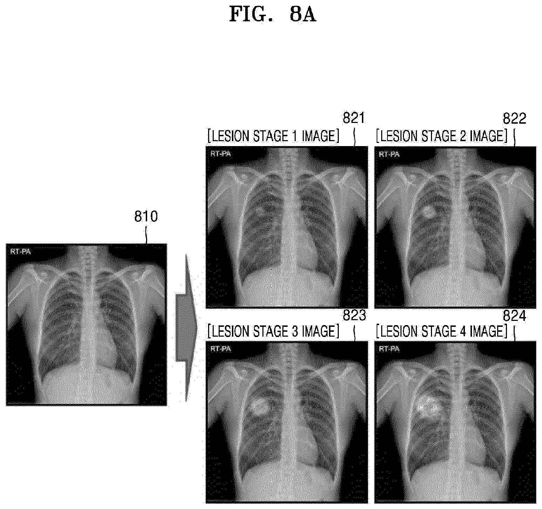

FIG. 8A is a view illustrating second medical images generated according to an embodiment.

The DNN 520 that is used in the medical image processing apparatus 300 may receive an actual medical image 810 representing the entire object, and may obtain at least one artificial medical image, namely, four artificial medical images 821, 822, 823, and 824, which are at least one second medical image corresponding to the actual medical image 810. As shown in FIG. 8A, the at least one artificial medical image output by the DNN 520 may include a lesion stage 1 image 821, a lesion stage 2 image 822, a lesion stage 3 image 823, and a lesion stage 4 image 824.

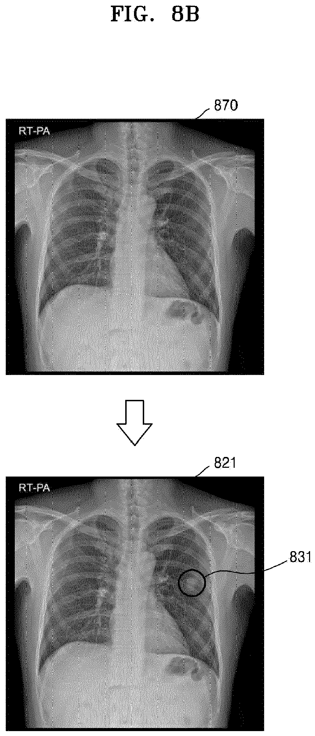

FIG. 8B is a view illustrating a second medical image generated according to an embodiment.

Referring to FIG. 8B, a second medical image described above with reference to FIG. 8A may be obtained by adding an artificial lesion included in the artificial lesion images of FIG. 6 to a medical image 870. The medical image 870 may be a general medical image having no lesions. Alternatively, the medical image 870 may be the actual medical image 810 of FIG. 8A. For example, a lesion stage 1 image 821 may be generated by overlapping or adding a stage 1 lesion 831 onto the medical image 870.

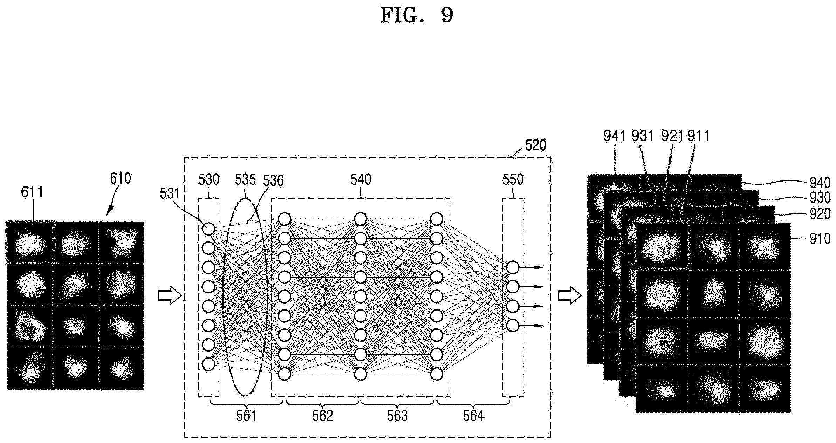

FIG. 9 is a view for describing generation of a second medical image according to an embodiment.

Referring to FIG. 9, the medical image processing apparatus 300 may generate a plurality of artificial lesion images 910, 920, 930, and 940 respectively corresponding to the plurality of actual lesion images 610, via an operation based on the DNN 520.

For example, the medical image processing apparatus 300 may input an actual lesion image 611 obtained at a first time point to the first neural network, which is the DNN 520, and perform an operation based on the DNN 520 to output one or more artificial lesion images 911, 921, 931, and 941 representing states or characteristics of a lesion included in the actual lesion image 611 at one or more time points different from the first time point. When a lesion at the first time point corresponds to a stage 0 lesion, the four artificial lesion images 911, 921, 931, and 941 output by the DNN 520 may be an image 911 representing a lesion in stage 1, an image 921 representing a lesion in stage 2, an image 931 representing a lesion in stage 3, and an image 941 representing a lesion in stage 4, respectively. The DNN 520 may also output information indicting the characteristics of a development aspect or change state of the lesion over time.

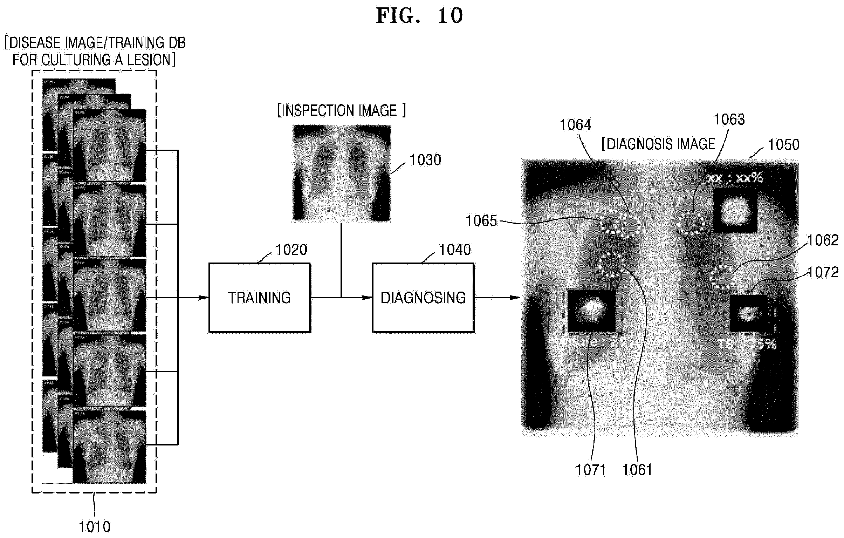

FIG. 10 is a diagram illustrating generation of diagnosis information according to an embodiment.

Various pieces of training data are needed to increase the accuracy of an inferring operation that is performed by the DNN 520. In other words, the accuracy of a result of inferring new input data may be increased by training the DNN 520 with various pieces of training data.

Accordingly, the medical image processing apparatus 300 according to an embodiment may train the plurality of second medical images obtained via the first neural network. The medical image processing apparatus 300 according to an embodiment may also train the plurality of actual medical images obtained by the data obtainer 310 of the medical image processing apparatus 300 via the first neural network. In other words, the medical image processing apparatus 300 may perform training based on more pieces of input data, in addition to the training of the plurality of actual medical images, in order to increase the operation accuracy of the first neural network. The medical image processing apparatus 300 may update the first neural network by adjusting the weighted values of the plurality of nodes that form the first neural network, based on a result of the training. The updated first neural network may be referred to as the second neural network.

When the first neural network is trained using the plurality of second medical images, which are artificially generated medical images, the operation accuracy of the first neural network may be increased. Accordingly, the second neural network may more accurately infer, for example, the characteristics of a certain lesion, a change aspect and form of the certain lesion over time, and the characteristics of the certain lesion over time.

Referring to FIG. 10, the medical image processing apparatus 300 may use at least one second medical image as a training DB for training the DNN 520 in order to infer a change state of the certain lesion over time. In FIG. 10, a plurality of actual medical images for use in training are referred to as disease images, and artificial medical images for use in training are referred to as a training DB. Accordingly, the medical image processing apparatus 300 may train a DNN by using a disease image/training DB 1010, as indicated by reference numeral 1020.

Because an actual medical image is used to train a conventional DNN, there are limitations with regard to the training DB. However, according to an embodiment of the present disclosure, the medical image processing apparatus 300 may expand the range of the training DB by including artificial medical images obtained by the medical image processing apparatus 300 in the training DB, thereby overcoming the limitation of the conventional training DB. Accordingly, the operation accuracy of a DNN may be increased.

The medical image processing apparatus 300 according to an embodiment may analyze a third medical image obtained by scanning an object of an examinee via the second neural network, and may obtain diagnosis information corresponding to the object of the examinee as a result of the analysis. The third medical image is shown as an inspection image 1030 in FIG. 10.

In detail, referring to FIG. 10, the medical image processing apparatus 300 may perform an operation by inputting the inspection image 1030 to the second neural network, which is a data recognition model established by training the actual medical images and the artificial medical images, as indicated by reference numeral 1020. The medical image processing apparatus 300 may obtain diagnosis information necessary for diagnosing the inspection image 1030, as indicated by reference numeral 1040.

The diagnosis information may include at least one of the type of disease having occurred in the object, the characteristics of the disease, the possibility that the disease may change or develop over time, the type of additional disease that may occur due to the previous disease, the characteristics of the additional disease, and the possibility that the additional disease changes or develops over time.

In FIG. 10, a diagnosis image 1050 is illustrated as the generated diagnosis information. In detail, the diagnosis image 1050 may represent a result of analyzing the inspection image 1030, detecting an abnormal part of an object from the inspection image 1030, and analyzing the characteristics of the detected abnormal part. The abnormal part refers to any body part that is not normal, and may include any body part having a lesion or any body part likely to have a lesion.

For example, when five abnormal parts 1061, 1062, 1063, 1064, and 1065 are detected from the diagnosis image 1050, pieces of analysis information respectively corresponding to the abnormal parts 1061, 1062, 1063, 1064, and 1065 may be represented on the corresponding abnormal parts 1061, 1062, 1063, 1064, and 1065. For example, when, as a result of analyzing the detected abnormal part 1061, the abnormal part 1061 is highly likely to be a nodule, the possibility of the abnormal part 1061 being a nodule (e.g., "Nodule: 89%") and a magnified image 1071 of the detected abnormal part 1061 may be overlaid on the diagnosis image 1050, as shown in FIG. 10. When another abnormal part 1062 is highly likely to be tuberculosis (TB), the possibility of the abnormal part 1062 being TB (e.g., "TB: 75%") and a magnified image 1072 of the detected abnormal part 1062 may be overlaid on the diagnosis image 1050, as shown in FIG. 10.

The display 350 of the medical image processing apparatus 300 may display diagnosis information output as a result of the operation based on the second neural network, for example, the diagnosis image 1050, under the control of the controller 320.

The controller 320 of the medical image processing apparatus 300 may transmit the diagnosis information output as a result of the operation based on the second neural network, for example, the diagnosis image 1050, to an external electronic apparatus via the communicator 315. For example, when the external electronic apparatus connected via the communicator 315 is a PACS server or a PACS viewer, the controller 320 may control the obtained diagnosis information to be transmitted to the PACS viewer. Accordingly, a doctor may more easily and more quickly diagnose an object by using the diagnosis information displayed on the PACS viewer.

As described above, the medical image processing apparatus 300 according to an embodiment may more accurately diagnose and predict a lesion or disease having occurred in an object of an examinee, by learning and/or predicting a variation in the lesion or disease over time by culturing the lesion or disease according to AI technology as a result of the operation based on the DNN 520.

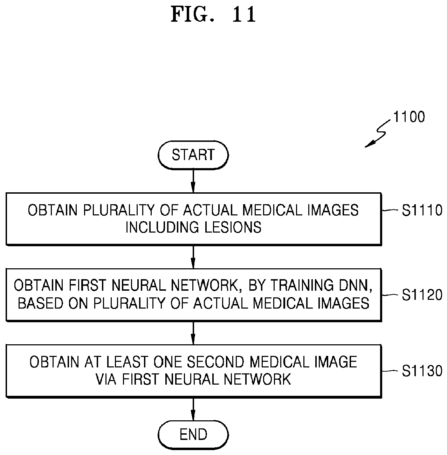

FIG. 11 is a flow chart of a medical image processing method 1100 according to an embodiment.

The medical image processing method 1100 according to an embodiment may be performed by the medical image processing apparatus 200 or 300 according to an embodiment. Accordingly, operations of the medical image processing method 1100 may be performed by components of the medical image processing apparatus 200 or 300, respectively, and the medical image processing method 1100 may include the same structural features as the medical image processing apparatus 200 or 300. Accordingly, descriptions of the medical image processing method 1100 that are the same as those made with reference to FIGS. 1 through 10 are not repeated herein.

The medical image processing method 1100 will now be described in detail with reference to the medical image processing apparatus 300 of FIG. 3.

Referring to FIG. 11, in the medical image processing method 1100 according to an embodiment, a plurality of actual medical images corresponding to a plurality of patients and including lesions are obtained, in operation S1110. Operation S1110 may be performed by the data obtainer 310.

Then, in operation S1120, a DNN may be trained based on the plurality of actual medical images obtained in operation S1110, to thereby obtain the first neural network for predicting a variation of a lesion over time. Operation S1120 may be performed in the controller 320. Alternatively, operation S1120 may be performed by the DNN processor 330. Alternatively, operation S1120 may be performed by the DNN processor 330 under the control of the controller 320.

Then, in operation S1130, at least one second medical image representing a state of a lesion included in a first medical image included in the plurality of actual medical images at at least one time point different from a first time point, which is a time point when the first medical image has been obtained, is obtained via the first neural network. Operation S1130 may be performed by the controller 320. Alternatively, operation S1130 may be performed by the DNN processor 330. Alternatively, operation S1130 may be performed by the DNN processor 330 under the control of the controller 320.

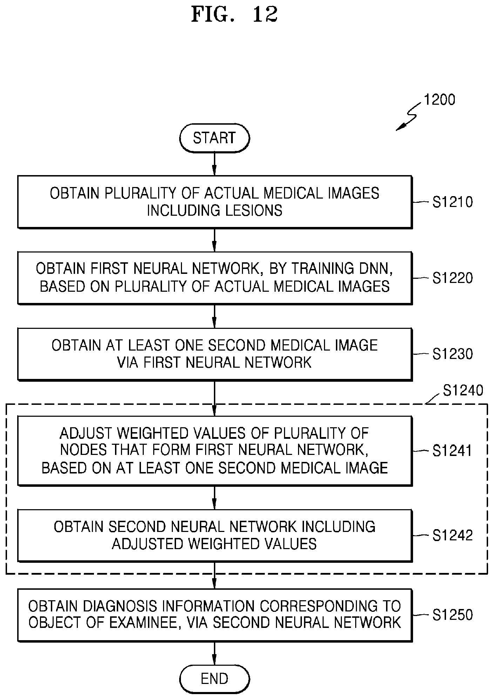

FIG. 12 is a flow chart of a medical image processing method 1200 according to another embodiment. Operations S1210, S1220 and S1230 of the medical image processing method 1200 of FIG. 12 may correspond to operations S1110, S1120 and S1130 of the medical image processing method 1100 of FIG. 11, respectively. Accordingly, descriptions of the medical image processing method 1200 that are the same as those of the medical image processing method 1100 and those made with reference to FIGS. 1 through 10 are not repeated herein.

The medical image processing method 1200 may further include operation S1240 and S1250, compared with the medical image processing method 1100.

Referring to FIG. 12, operation S1230 may be followed by operation S1240 of establishing the second neural network by training the at least one second medical image. Operation S1240 may be performed by the controller 320. Alternatively, operation S1240 may be performed by the DNN processor 330. Alternatively, operation S1240 may be performed by the DNN processor 330 under the control of the controller 320.

In detail, in operation S1241, the first neural network may be trained based on the at least one second medical image, to thereby adjust the weighted values of the plurality of nodes that form the first neural network. In operation S1242, a second neural network including the adjusted weighted values may be obtained. In other words, the second neural network may be obtained by correcting or updating the first neural network.

Then, in operation S1250, diagnosis information corresponding to an object of an examinee may be obtained via the second neural network. Operation S1250 may be performed by the controller 320. Alternatively, operation S1250 may be performed by the DNN processor 330. Alternatively, operation S1250 may be performed by the DNN processor 330 under the control of the controller 320.

In detail, in operation S1250, a third medical image obtained by scanning the object of the examinee, for example, the inspection image 1030, may be analyzed via the second neural network, and diagnosis information corresponding to the object of the examinee, for example, the diagnosis image 1050, may be obtained as a result of the analysis.

In a medical image processing method according to an embodiment and a medical image processing apparatus performing the same, a user, such as a doctor, is able to easily predict or ascertain a development aspect of the lesion at a future time point subsequent to a time point when an actual medical image has been obtained, via a second medical image, in addition to the state of a lesion at the time point when the actual medical image has been obtained.

In addition, in the medical image processing method according to an embodiment and the medical image processing apparatus performing the same, a DNN is trained using a plurality of artificially obtained second medical images, thereby increasing the diversity of training data and overcoming the limitations of the training data.

Moreover, in the medical image processing method according to an embodiment and the medical image processing apparatus performing the same, the accuracy of the DNN may be improved and increased due to the training of the DNN by using the plurality of artificially obtained second medical images. This may lead to an improvement in the accuracy of diagnosis information of an inspection image that is subsequently obtained.

Embodiments may be implemented through non-transitory computer-readable recording media having recorded thereon computer-executable instructions and data. The instructions may be stored in the form of program code, and when executed by a processor, generate a predetermined program module to perform a specific operation. Furthermore, when being executed by the processor, commands corresponding to the instructions may perform specific operations according to the embodiments.

## Claims

1. A medical image processing method comprising:
obtaining a plurality of actual medical images (510; 810) corresponding to a plurality of patients and including lesions (511) (S1110);
obtaining a first neural network (520), which is a trained deep neural network (520) based on the plurality of actual medical images (510; 810), for predicting a variation in a lesion over time, a lesion being included in a first medical image of the plurality of actual medical images, wherein the first medical image is obtained at a first time point (S1120); and
generating by inputting the first medical image to the first neural network (520), a second medical image which is an artificial lesion image (570) representing a state of the lesion at a second time point different from the first time point (S1130).

2. The medical image processing method of claim 1 , wherein the first neural network (520) predicts at least one of (i) a developing or changing form of the lesion (511) included in each of the plurality of actual medical images (510; 810) over time, (ii) a possibility that an additional disease occurs due to the lesion (511), and (iii) a developing or changing form of the additional disease over time due to the lesion (511), and outputs an artificial medical image (721, 722, 723, 724; 821, 822, 823, 824) including a result of the predicting as the second medical image (570).

3. The medical image processing method of any of the previous claims, wherein the state of the lesion (511) comprises at least one of a generation time of the lesion (511), a developing or changing form of the lesion (511), a possibility that an additional disease occurs due to the lesion (511), and a developing or changing form of the additional disease due to the lesion (511).

4. The medical image processing method of any of the previous claims, wherein:
the first neural network (S1241) is trained based on the second medical image, to adjust weighted values of a plurality of nodes that form the first neural network, and
the method further comprising:
obtaining a second neural network (S1242) comprising the adjusted weighted values.

5. The medical image processing method of claim 4, further comprising analyzing a third medical image obtained by scanning an object of an examinee via the second neural network, and obtaining diagnosis information corresponding to the object of the examinee as a result of the analysis (S1250).

6. The medical image processing method of claim 5, wherein the diagnosis information comprises at least one of a type of a disease having occurred in the object, characteristics of the disease, a possibility that the disease changes or develops over time, a type of an additional disease occurring due to the disease, characteristics of the additional disease, and a changing or developing state of the additional disease over time.

7. The medical image processing method of any of the previous claims, further comprising displaying a screen image including the second medical image.

8. The medical image processing method of claim 1, wherein the second medical image is an X-ray image representing an object including the lesion (570; 821, 822, 823, 824).

9. The medical image processing method of claim 1, wherein the second medical image is a lesion image (721, 722, 723, 724) representing the state of the lesion at the second time point different from the first time point.

10. A medical image processing apparatus (200) comprising:
a data obtainer (210; 310; 410-1) configured to obtain a plurality of actual medical images (510; 810) corresponding to a plurality of patients and including lesions (511); and
a controller (220) configured to:
obtain a first neural network (520), which is a trained deep neural network based on the plurality of actual medical images (510; 810), for predicting a variation in a lesion over time , a lesion (511) being included in a first medical image (510) of the plurality of actual medical images (810), wherein the first medical image (510) is obtained at a first time point, and
generate, by inputting the first medical image to the first neural network (520), a second medical image which is an artificial lesion image (570) representing a state of the lesion at a second time point different from the first time point.

11. The medical image processing apparatus (200) of claim 10, wherein the first neural network (520) predicts at least one of (i) a developing or changing form of the lesion (511) included in each of the plurality of actual medical images (810) over time, (ii) a possibility that an additional disease occurs due to the lesion (511), and (iii) a developing or changing form of the additional disease over time due to the lesion (511), and outputs an artificial medical image (721, 722, 723, 724; 821, 822, 823, 824)including a result of the predicting as the second medical image (570).

12. The medical image processing apparatus (200) of claim 10 or 11, wherein the state of the lesion comprises at least one of a generation time of the lesion (511), a developing or changing form of the lesion (511), a possibility that an additional disease occurs due to the lesion (511), characteristics of the additional disease, and a developing or changing form of the additional disease due to the lesion (511).

13. A non-transitory computer-readable recording medium having recorded thereon instructions which, when executed by a processor, cause the processor to perform operations comprising:
obtaining a plurality of actual medical images (510; 810) corresponding to a plurality of patients and including lesions (511);
obtaining a first neural network (520), which is a trained deep neural network (520) based on the plurality of actual medical images (510; 810), for predicting a variation in a lesion (511) over time, a lesion (511) being included in a first medical image (510) of the plurality of actual medical images (810), wherein the first medical image (510) is obtained at a first time point; and
generating, by inputting the first medical image to the first neural network (520), a second medical image which is an artificial lesion image (721, 722, 723, 724; 821, 822, 823, 824) representing a state of the lesion (511) at a second time point different from the first time point.

## Patentansprüche

1. Verfahren zur Verarbeitung medizinischer Bilder, das Folgendes umfasst:
Erhalten einer Vielzahl von tatsächlichen medizinischen Bildern (510; 810), die einer Vielzahl von Patienten entsprechen und Läsionen (511) enthalten (S1110);
Erhalten eines ersten neuronalen Netzwerks (520), bei dem es sich um ein trainiertes tiefes neuronales Netzwerk (520) handelt, basierend auf der Vielzahl von tatsächlichen medizinischen Bildern (510; 810), um eine im Laufe der Zeit bei einer Läsion auftretende Variation vorherzusagen, wobei eine Läsion in einem ersten medizinischen Bild aus der Vielzahl von tatsächlichen medizinischen Bildern enthalten ist, wobei das erste medizinische Bild an einem ersten Zeitpunkt erhalten wird (S1120); und
durch Eingeben des ersten medizinischen Bildes in das erste neuronale Netzwerk (520) Erzeugen eines zweiten medizinischen Bildes, bei dem es sich um ein künstliches Läsionsbild (570) handelt, das einen Zustand der Läsion an einem Zeitpunkt darstellt, der sich von dem ersten Zeitpunkt unterscheidet (S1130).

2. Verfahren zur Verarbeitung medizinischer Bilder nach Anspruch 1, wobei das erste neuronale Netzwerk (520) wenigstens eines von Folgendem vorhersagt: (i) eine Entwicklung oder Veränderung der Form der Läsion (511), die in jedem aus der Vielzahl von tatsächlichen medizinischen Bildern (510; 810) enthalten ist, im Laufe der Zeit, (ii) eine Möglichkeit, dass eine zusätzliche Krankheit wegen der Läsion (511) auftritt, und (iii) eine Entwicklung oder Veränderung der Form der zusätzlichen Krankheit wegen der Läsion (511) im Laufe der Zeit, und ein das Ergebnis der Vorhersage enthaltendes künstliches medizinisches Bild (721, 722, 723, 724; 821, 822, 823, 824) als das zweite medizinische Bild (570) ausgibt.

3. Verfahren zur Verarbeitung medizinischer Bilder nach einem der vorhergehenden Ansprüche, wobei der Zustand der Läsion (511) wenigstens eine der Folgenden umfasst: eine Erzeugungszeit der Läsion (511), eine Entwicklung oder Veränderung der Form der Läsion (511), eine Möglichkeit, dass eine zusätzliche Krankheit wegen der Läsion (511) auftritt, und eine Entwicklung oder Veränderung der Form der zusätzlichen Krankheit wegen der Läsion (511)

4. Verfahren zur Verarbeitung medizinischer Bilder nach einem der vorhergehenden Ansprüche, wobei:
das erste neuronale Netzwerk (S1241) basierend auf dem zweiten medizinischen Bild trainiert wird, um gewichtete Werte einer Vielzahl von das erste neuronale Netzwerk bildenden Knoten anzupassen, und
das Verfahren weiterhin Folgendes umfasst:
Erhalten eines zweiten neuronalen Netzwerks (S1242), das die angepassten gewichteten Werte umfasst.

5. Verfahren zur Verarbeitung medizinischer Bilder nach Anspruch 4, das weiterhin Folgendes umfasst: Analysieren eines dritten medizinischen Bildes, das durch Abtasten eines Objektes einer zu untersuchenden Person über das zweite neuronale Netzwerk erhalten wird, und Erhalten von Diagnoseinformationen, die dem Objekt der zu untersuchenden Person entsprechen, als ein Ergebnis der Analyse (S1250).

6. Verfahren zur Verarbeitung medizinischer Bilder nach Anspruch 5, wobei die Diagnoseinformationen wenigstens eines von Folgendem umfassen: eine Art von Krankheit, die in dem Objekt aufgetreten ist, Eigenschaften der Krankheit, eine Möglichkeit, dass die Krankheit sich im Laufe der Zeit verändert oder entwickelt, eine Art einer zusätzlichen Krankheit, die wegen der Krankheit auftritt, Eigenschaften der zusätzlichen Krankheit, und einen Veränderungs- oder Entwicklungszustand der zusätzlichen Krankheit im Laufe der Zeit.

7. Verfahren zur Verarbeitung medizinischer Bilder nach einem der vorhergehenden Ansprüche, das weiterhin das Anzeigen eines das zweite medizinische Bild enthaltenden Bildschirmbildes umfasst.

8. Verfahren zur Verarbeitung medizinischer Bilder nach Anspruch 1, wobei es sich bei dem zweiten medizinischen Bild um ein Röntgenbild handelt, das ein die Läsion (570; 821, 822, 823, 824) enthaltendes Objekt darstellt.

9. Verfahren zur Verarbeitung medizinischer Bilder nach Anspruch 1, wobei es sich bei dem zweiten medizinischen Bild um ein Läsionsbild (721, 722, 723, 724) handelt, das den Zustand der Läsion an dem zweiten Zeitpunkt, der sich von dem ersten Zeitpunkt unterscheidet, darstellt.

10. Vorrichtung (200) zur Verarbeitung medizinischer Bilder, die Folgendes umfasst:
eine Datenerhaltungsvorrichtung (210; 310, 410-1), die zum Erhalten einer Vielzahl von tatsächlichen medizinischen Bildern (510; 810), die einer Vielzahl von Patienten entsprechen und Läsionen (511) enthalten, konfiguriert ist;
eine Steuerung (220), die zu Folgendem konfiguriert ist:
Erhalten eines ersten neuronalen Netzwerks (520), bei dem es sich um ein trainiertes tiefes neuronales Netzwerk handelt, basierend auf der Vielzahl von tatsächlichen medizinischen Bildern (510; 810), um eine im Laufe der Zeit bei einer Läsion (511) auftretende Variation vorherzusagen, wobei eine Läsion in einem ersten medizinischen Bild (510) aus der Vielzahl von tatsächlichen medizinischen Bildern (810) enthalten ist, wobei das erste medizinische Bild (510) an einem ersten Zeitpunkt erhalten wird; und
durch Eingeben des ersten medizinischen Bildes in das erste neuronale Netzwerk (520) Erzeugen eines zweiten medizinischen Bildes, bei dem es sich um ein künstliches Läsionsbild (570) handelt, das einen Zustand der Läsion an einem Zeitpunkt darstellt, der sich von dem ersten Zeitpunkt unterscheidet.

11. Vorrichtung (200) zur Verarbeitung medizinischer Bilder nach Anspruch 10, wobei das erste neuronale Netzwerk (520) wenigstens eines von Folgendem vorhersagt: (i) eine Entwicklung oder Veränderung der Form der Läsion (511), die in jedem aus der Vielzahl von tatsächlichen medizinischen Bildern (810) enthalten ist, im Laufe der Zeit, (ii) eine Möglichkeit, dass eine zusätzliche Krankheit wegen der Läsion (511) auftritt, und (iii) eine Entwicklung oder Veränderung der Form der zusätzlichen Krankheit wegen der Läsion (511) im Laufe der Zeit, und ein das Ergebnis der Vorhersage enthaltendes künstliches medizinisches Bild (721, 722, 723, 724; 821, 822, 823, 824) als das zweite medizinische Bild (570) ausgibt.

12. Vorrichtung (200) zur Verarbeitung medizinischer Bilder nach Anspruch 10 oder 11, wobei der Zustand der Läsion wenigstens eines von Folgendem umfasst: eine Erzeugungszeit der Läsion (511), eine Entwicklung oder Veränderung der Form der Läsion (511), eine Möglichkeit, dass eine zusätzliche Krankheit wegen der Läsion (511) auftritt, Eigenschaften der zusätzlichen Krankheit, und eine Entwicklung oder Veränderung der Form der zusätzlichen Krankheit wegen der Läsion (511).

13. Nicht flüchtiges, computerlesbares Aufzeichnungsmedium, in dem Anweisungen aufgezeichnet sind, die, wenn sie von einem Prozessor ausgeführt werden, den Prozessor veranlassen, Vorgänge durchzuführen, die Folgendes umfassen:
Erhalten einer Vielzahl von tatsächlichen medizinischen Bildern (510; 810), die einer Vielzahl von Patienten entsprechen und Läsionen (511) enthalten;
Erhalten eines ersten neuronalen Netzwerks (520), bei dem es sich um ein trainiertes tiefes neuronales Netzwerk (520) handelt, basierend auf der Vielzahl von tatsächlichen medizinischen Bildern (510; 810), um eine im Laufe der Zeit bei einer Läsion (511) auftretende Variation vorherzusagen, wobei eine Läsion (511) in einem ersten medizinischen Bild (510) aus der Vielzahl von tatsächlichen medizinischen Bildern (810) enthalten ist, wobei das erste medizinische Bild (510) an einem ersten Zeitpunkt erhalten wird; und
durch Eingeben des ersten medizinischen Bildes in das erste neuronale Netzwerk (520) Erzeugen eines zweiten medizinischen Bildes, bei dem es sich um ein künstliches Läsionsbild (721, 722, 723, 724; 821, 822, 823, 824) handelt, das einen Zustand der Läsion (511) an einem Zeitpunkt darstellt, der sich von dem ersten Zeitpunkt unterscheidet.

## Revendications

1. Procédé de traitement d'image médicale, comprenant :
l'obtention d'une pluralité d'images médicales effectives (510 ; 810) correspondant à une pluralité de patients et comprenant des lésions (511) (S1110),
l'obtention d'un premier réseau neuronal (520), consistant en un réseau neuronal profond entraîné (520) reposant sur la pluralité d'images médicales effectives (510 ; 810), pour prédire un changement concernant une lésion dans le temps, une lésion étant présente dans une première image médicale de la pluralité d'images médicales effectives, ladite première image médicale étant obtenue à un premier instant (S1120), et
la génération, grâce à l'introduction de la première image médicale dans le premier réseau neuronal (520), d'une deuxième image médicale consistant en une image de lésion artificielle (570) représentant un état de la lésion à un deuxième instant différent du premier instant (S1130).

2. Procédé de traitement d'image médicale selon la revendication 1, dans lequel le premier réseau neuronal (520) prédit (i) une forme de développement ou d'évolution de la lésion (511) présente dans chacune des images de la pluralité d'images médicales effectives (510 ; 810) dans le temps, (ii) une possibilité de survenue de maladie supplémentaire du fait de la lésion (511) et/ou (iii) une forme de développement ou d'évolution de la maladie supplémentaire dans le temps du fait de la lésion (511), et produit une image médicale artificielle (721, 722, 723, 724 ; 821, 822, 823, 824) comprenant un résultat de la prédiction sous la forme de la deuxième image médicale (570).

3. Procédé de traitement d'image médicale selon l'une quelconque des revendications précédentes, dans lequel l'état de la lésion (511) comprend le moment de génération de la lésion (511), une forme de développement ou d'évolution de la lésion (511), une possibilité de survenue de maladie supplémentaire du fait de la lésion (511) et/ou une forme de développement ou d'évolution de la maladie supplémentaire due à la lésion (511).

4. Procédé de traitement d'image médicale selon l'une quelconque des revendications précédentes, dans lequel :
le premier réseau neuronal (S1241) est entraîné en fonction de la deuxième image médicale, pour ajuster des valeurs pondérées d'une pluralité de nœuds formant le premier réseau neuronal,
le procédé comprenant en outre :
l'obtention d'un deuxième réseau neuronal (S1242) comprenant les valeurs pondérées ajustées.

5. Procédé de traitement d'image médicale selon la revendication 4, comprenant en outre l'analyse d'une troisième image médicale obtenue par balayage d'un objet d'une personne examinée, par le biais du deuxième réseau neuronal, et l'obtention d'informations de diagnostic correspondant à l'objet de la personne examinée, en résultat de l'analyse (S1250).

6. Procédé de traitement d'image médicale selon la revendication 5, dans lequel les informations de diagnostic comprennent le type d'une maladie étant survenue au sein de l'objet, des caractéristiques de la maladie, la possibilité que la maladie évolue ou se développe dans le temps, le type d'une maladie supplémentaire survenant du fait de la maladie, des caractéristiques de la maladie supplémentaire et/ou un état d'évolution et de développement de la maladie supplémentaire dans le temps.

7. Procédé de traitement d'image médicale selon l'une quelconque des revendications précédentes, comprenant en outre l'affichage d'une image d'écran comprenant la deuxième image médicale.

8. Procédé de traitement d'image médicale selon la revendication 1, dans lequel la deuxième image médicale est une image radiologique représentant un objet comprenant la lésion (570 ; 821, 822, 823, 824).

9. Procédé de traitement d'image médicale selon la revendication 1, dans lequel la deuxième image médicale est une image de lésion (721, 722, 723, 724) représentant l'état de la lésion au deuxième instant différent du premier instant.

10. Appareil de traitement d'image médicale (200) comprenant :
un organe d'obtention de données (210 ; 310, 410-1) conçu pour obtenir une pluralité d'images médicales effectives (510 ; 810) correspondant à une pluralité de patients et comprenant des lésions (511) ; et
un organe de commande (220) conçu pour :
obtenir un premier réseau neuronal (520), consistant en un réseau neuronal profond entraîné reposant sur la pluralité d'images médicales effectives (510 ; 810), pour prédire un changement concernant une lésion dans le temps, une lésion (511) étant présente dans une première image médicale (510) de la pluralité d'images médicales effectives (810), ladite première image médicale (510) étant obtenue à un premier instant, et
générer, grâce à l'introduction de la première image médicale dans le premier réseau neuronal (520), une deuxième image médicale consistant en une image de lésion artificielle (570) représentant un état de la lésion à un deuxième instant différent du premier instant.

11. Appareil de traitement d'image médicale (200) selon la revendication 10, dans lequel le premier réseau neuronal (520) prédit (i) une forme de développement ou d'évolution de la lésion (511) présente dans chacune des images de la pluralité d'images médicales effectives (810) dans le temps, (ii) une possibilité de survenue de maladie supplémentaire du fait de la lésion (511) et/ou (iii) une forme de développement ou d'évolution de la maladie supplémentaire dans le temps du fait de la lésion (511), et produit une image médicale artificielle (721, 722, 723, 724 ; 821, 822, 823, 824) comprenant un résultat de la prédiction sous la forme de la deuxième image médicale (570).

12. Appareil de traitement d'image médicale selon la revendication 10 ou 11, dans lequel l'état de la lésion comprend le moment de génération de la lésion (511), une forme de développement ou d'évolution de la lésion (511), une possibilité de survenue de maladie supplémentaire du fait de la lésion (511), des caractéristiques de la maladie supplémentaire, et/ou une forme de développement ou d'évolution de la maladie supplémentaire due à la lésion (511).

13. Support d'enregistrement non transitoire lisible par ordinateur sur lequel sont enregistrées des instructions qui, lorsqu'elles sont exécutées par un processeur, amènent le processeur à effectuer les opérations comprenant :
l'obtention d'une pluralité d'images médicales effectives (510 ; 810) correspondant à une pluralité de patients et comprenant des lésions (511),
l'obtention d'un premier réseau neuronal (520), consistant en un réseau neuronal profond entraîné (520) reposant sur la pluralité d'images médicales effectives (510 ; 810), pour prédire un changement concernant une lésion dans le temps, une lésion (511) étant présente dans une première image médicale (510) de la pluralité d'images médicales effectives (810), ladite première image médicale (510) étant obtenue à un premier instant, et
la génération, grâce à l'introduction de la première image médicale dans le premier réseau neuronal (520), d'une deuxième image médicale consistant en une image de lésion artificielle (721, 722, 723, 724 ; 821, 822, 823, 824) représentant un état de la lésion (511) à un deuxième instant différent du premier instant.
